# EUROPEAN PATENT APPLICATION

(11) **EP 3 904 499 A1**
(43) Date of publication of application: **03.11.2021**
(21) Application number: 19902062.9
(22) Date of filing: 15.11.2019
(51) Int. Cl.: C12N 1/14, C12N 1/02, C12P 13/04, C12R 1/645

(54) **STRAIN PRODUCING ERGOTHIONEINE AND SCREENING METHOD THEREOF**

(30) Priority: 26.12.2018 CN 201811605008
(71) Applicant: Bloomage Biotechnology Corporation Limited, Jinan, Shandong 250101 (CN); Shandong Bloomage Hyinc Biopharm Corporation Limited, Jinan, Shandong 250000 (CN)
(72) Inventor: WEI, Yujie, Jinan, Shandong 250101 (CN); LU, Zhen, Jinan, Shandong 250101 (CN); JIA, Yuqian, Jinan, Shandong 250101 (CN); SUN, Yuanjun, Jinan, Shandong 250101 (CN); CHEN, Wenwen, Jinan, Shandong 250101 (CN); SHI, Yanli, Jinan, Shandong 250101 (CN); LUAN, Yihong, Jinan, Shandong 250101 (CN); GUO, Xueping, Jinan, Shandong 250101 (CN)
(74) Representative: Bianchetti & Minoja SRL
(86) International application number: PCT/CN2019/118948
(87) International publication number: WO 2020/134689

(57) **Abstract**

The present invention belongs to the field of microbial technology, and specifically relates to a strain of *Hericium erinaceus* HT-3, with a deposit number of CCTCC No: M 2018567. The present invention also relates to a screening method for *Hericium erinaceus* HT-3. The screening method comprises the steps of purifying the strain of *Hericium erinaceus* from a tissue block, fermenting and culturing the strain, soak extracting ergothioneine from the mycelium cells in the fermentation broth, detecting the ergothioine content in the fermentation broth. The *Hericium erinaceus* strain screened according to the present invention has high ergothioneine yield, and the screening method is simple in process, easy to be operated, and low in production cost.

Sequence Listing

<110>  BLOOMAGE BIOTECHNOLOGY CORPORATION LIMITED
       SHANDONG BLOOMAGE HYINC BIOPHARM CORPORATION LIMITED

<120>  STRAIN PRODUCING ERGOTHIONEINE AND METHOD FOR SCREENING THE SAME

<130>  PB00324

<141>  2018-12-26

<160>  2

<170>  SIPOSequenceListing 1.0

<210>  1
<211>  1316
<212>  DNA/RNA
<213>  Hericium erinaceus

<400>  1
ttgtactgtg aaactgcgaa tggctcatta aatcagttat agtttatttg atggtacctt      60
gctacatgga taactgtggt aattctagag ctaatacatg caattaagcc ccgacttccg     120
gaaggggtgt atttattaga taaaaaacca acgcggctcg ccgctccttt ggtgattcat     180
aataacttct cgaatcgcat ggccttgtgc cggcgatgct tcattcaaat atctgcccta     240
tcaactttcg atggtaggat agaggcctac catggtttca acgggtaacg gggaataagg     300
gttcgattcc ggagagggag cctgagaaac ggctaccaca tccaaggaag gcagcaggcg     360
cgcaaattac ccaatcccga cacggggagg tagtgacaat aaataacaat atagggctct     420
tttgggtctt ataattggaa tgagtacaat ttaaatccct taacgaggaa caattggagg     480
gcaagtctgg tgccagcagc cgcggtaatt ccagctccaa tagcgtatat taaagttgtt     540
gcagttaaaa agctcgtagt tgaacttcag gcctggccgg gcggtctgcc taacggtatg     600
tactgtctgg ccgggcctta cctcctggtg agccggcatg cccttcactg ggtgtgtcgg     660
ggaaccagga cttttacctt gagaaaatta gagtgttcaa agcaggccta tgcccgaata     720
cattagcatg gaataataaa ataggacgtg cggttctatt ttgttggttt ctagaatcgc     780
cgtaatgatt aatagggata gttgggggca ttagtattgc gttgctagag gtgaaattct     840
tggatttacg caagactaac tactgcgaaa gcatttgcca aggatgtttt cattaatcaa     900
gaacgaaggt taggggatcg aaaacgatca gataccgttg tagtcttaac agtaaactat     960
gccgactagg gatcgggcga cctcaattta atgtgtcgct cggcacctta cgagaaatca    1020
aagtctttgg gttctggggg gagtatggtc gcaaggctga aacttaaagg aattgacgga    1080
agggcaccac caggagtgga gcctgcggct taatttgact caacacgggg aaactcacca    1140
ggtccagaca taactaggat tgacagattg atagctcttt cttgatttta tgggtggtgg    1200
tgcatggccg ttcttagttg gtggagtgat ttgtctggtt aattccgata acgaacgaga    1260
ccttaacctg ctaaatagcc cggccggctt ttgctggtcg ctggcttctt agaggg        1316

<210>  2
<211>  605
<212>  DNA/RNA
<213>  Hericium erinaceus

<400>  2
tgcggaagga tcattaatga atttgaaagg agttgttgct ggcctgaaac ccaggcatgt      60
gcacgctcca atctcatcca tcttacacct gtgcaccctt gcgtgggtcc gtcggctttg     120
cggtcgatgg gcttgcgttt ttcataaact cttatgtatg taacagaatg tcataatgct     180
ataaacgcat cttatacaac tttcaacaac ggatctcttg gctctcgcat cgatgaagaa     240
cgcagcgaaa tgcgataagt aatgtgaatt gcagaattca gtgaatcatc gaatctttga     300
acgcaccttg cgccccttgg tattccgagg ggcacgcctg ttcgagtgtc gtgaaattct     360
caactcaatc ctcttgttat gagagggctg ggcttggact tggaggtctt gccggtgctc     420
cctcgggaag tcggctcctc ttgaatgcat gagtggatcc cttttgtagg gtttgccctt     480
ggtgtgataa ttatctacgc cgcgggtagc cttgcgttgg tctgcttcta accgtcttcg     540
gacaactttc atctcaactt gacctcgaat caggcgggac tacccgctga acttaagcat     600
atcat                                                                605

## Description

### FIELD OF THE INVENTION

The present invention belongs to the field of microbial technology, and specifically relates to a strain of *Hericium erinaceus* HT-3 and a method for screening the same.

### BACKGROUND OF THE INVENTION

Ergothioneine, a scientific name of which is 2-mercapto-L-histidine trimethyl-, inner salt, is a natural 2-thioimidazole amino acid. Ergothioneine in the dissolved state has two tautomeric isomers, thiol and thione, and it mainly exists in the form of thione under physiological pH conditions. Ergothioneine was first isolated by Tanret C from ergot (a sclerotium formed by a fungus that parasitizes rye, a gramineous plant) in 1909. The pure product is white crystals, has good water solubility with up to 0.9 mol/L at room temperature, and is quite stable under physiological pH and under strong alkaline conditions.

Ergothioneine has unique physiological functions, such as anti-oxidation, scavenging free radicals, chelating metal ions, protection against UV radiation damage, inhibiting cancers, etc., and in some aspects, it is superior over natural antioxidants such as glutathione.

Ergothioneine is present in various plants and animals. However, animals cannot synthesize ergothioneine by themselves and can only get it from foods. Many microorganisms such as fungi and actinomycetes can synthesize ergothioneine, but bacteria cannot synthesize this compound inside. So far, more studies have been performed on the production of ergothioneine by utilizing fungi.

Chinese patent application CN103184246A, "Method for biosynthetic preparation of ergothioneine", synthesizes ergothioneine by inoculation and fermentation of mycelium of macrofungi (*Lepista sordida, Pleurotus plumonarius,* and wild *Pleurotus sapidus*)*,* wherein, the highest content of ergothioneine in the fermentation broth was 51 mg/L for Lepista sordida, 48 mg/L for *Pleurotus plumonarius,* and 37 mg/L for wild *Pleurotus sapidus.* The ergothioneine yield is still not high and the growth cycle of the fungi is long.

Chinese patent application CN103734022A, "Strain for producing ergothioneine and methods for producing ergothioneine", discloses the steps of synthesizing ergothioneine by fermentation of *Pleurotus ostreatus* and extraction of ergothioneine from mycelium cells, but does not disclose a method for screening *Pleurotus ostreatus* strains.

Chinese patent application CN102978121A, "Method for producing ergothioneine by microorganism transformation", discloses the production of ergothioneine by transformation of microorganism *Lepista caespitosa*. However, the biotransformation affects the viability of fungal cells, and the transformation efficiency is low.

*Hericium erinaceus*, also known as Lion's Mane Mushroom, is named after its shape resembling hericium. The hyphae of *Hericium erinaceus* have thin cells walls, with septa and clamp connection. The fruiting bodies are clump, flat-hemisphere or head shaped, with hairy fleshy spines on the surface of pileus. The fungus is white when fresh, and from light yellow to light brown when dried. It has a narrow base or slightly short stalk, and an enlarged upper part with a diameter of 3.5-10 cm. It looks like golden hericium from a distance, so it is called *"Hericium erinaceus". Hericium erinaceus* is delicious, the mushroom body is fresh, tender, and fragrant, and is known as "vegetarian meat dish". *Hericium erinaceus* is not only delicious, but also contains a variety of amino acids, of which 8 are necessary for human body, and it is also rich in polysaccharides, various vitamins, inorganic salts, peptides and unsaturated fatty acids. *Hericium erinaceus* is easy to be fermented and cultivated, grows fast, and has a short growth cycle and a high yield.

### SUMMARY OF THE INVENTION

In order to solve the problem in the prior art, the present invention provides a strain of *Hericium erinaceus* HT-3, which was deposited at the China Center for Type Culture Collection (CCTCC) on August 23, 2018, and the deposit number is CCTCC No: M 2018567. The present invention also provides a composition comprising *Hericium erinaceus* and fermentation broth, and a method for screening *Hericium erinaceus.* The present invention includes embodiments as follows:
1. A *Hericium erinaceus* fungus, with deposit number CCTCC No: M 2018567.
2. The *Hericium erinaceus* fungus according to Item 1, which has an 18s rRNA gene sequence as shown in SEQ ID NO: 1 (TTGTACTGTGAAACTGCGAATGGCTCATTAAATCAGTTATAGTTTATTT GATGGTACCTTGCTACATGGATAACTGTGGTAATTCTAGAGCTAATACAT GCAATTAAGCCCCGACTTCCGGAAGGGGTGTATTTATTAGATAAAAAAC CAACGCGGCTCGCCGCTCCTTTGGTGATTCATAATAACTTCTCGAATCG CATGGCCTTGTGCCGGCGATGCTTCATTCAAATATCTGCCCTATCAACTT TCGATGGTAGGATAGAGGCCTACCATGGTTTCAACGGGTAACGGGGAA TAAGGGTTCGATTCCGGAGAGGGAGCCTGAGAAACGGCTACCACATCC AAGGAAGGCAGCAGGCGCGCAAATTACCCAATCCCGACACGGGGAGG TAGTGACAATAAATAACAATATAGGGCTCTTTTGGGTCTTATAATTGGAA TGAGTACAATTTAAATCCCTTAACGAGGAACAATTGGAGGGCAAGTCT GGTGCCAGCAGCCGCGGTAATTCCAGCTCCAATAGCGTATATTAAAGTT GTTGCAGTTAAAAAGCTCGTAGTTGAACTTCAGGCCTGGCCGGGCGGT CTGCCTAACGGTATGTACTGTCTGGCCGGGCCTTACCTCCTGGTGAGCC GGCATGCCCTTCACTGGGTGTGTCGGGGAACCAGGACTTTTACCTTGA GAAAATTAGAGTGTTCAAAGCAGGCCTATGCCCGAATACATTAGCATGG AATAATAAAATAGGACGTGCGGTTCTATTTTGTTGGTTTCTAGAATCGCC GTAATGATTAATAGGGATAGTTGGGGGCATTAGTATTGCGTTGCTAGAG GTGAAATTCTTGGATTTACGCAAGACTAACTACTGCGAAAGCATTTGCC AAGGATGTTTTCATTAATCAAGAACGAAGGTTAGGGGATCGAAAACGA TCAGATACCGTTGTAGTCTTAACAGTAAACTATGCCGACTAGGGATCGG GCGACCTCAATTTAATGTGTCGCTCGGCACCTTACGAGAAATCAAAGT CTTTGGGTTCTGGGGGGAGTATGGTCGCAAGGCTGAAACTTAAAGGAA TTGACGGAAGGGCACCACCAGGAGTGGAGCCTGCGGCTTAATTTGACT CAACACGGGGAAACTCACCAGGTCCAGACATAACTAGGATTGACAGAT TGATAGCTCTTTCTTGATTTTATGGGTGGTGGTGCATGGCCGTTCTTAGT TGGTGGAGTGATTTGTCTGGTTAATTCCGATAACGAACGAGACCTTAAC CTGCTAAATAGCCCGGCCGGCTTTTGCTGGTCGCTGGCTTCTTAGAGGG ).
3. The *Hericium erinaceus* fungus according to Item 1, which has an ITS sequence as shown in SEQ ID NO: 2 (TGCGGAAGGATCATTAATGAATTTGAAAGGAGTTGTTGCTGGCCTGAA ACCCAGGCATGTGCACGCTCCAATCTCATCCATCTTACACCTGTGCACC CTTGCGTGGGTCCGTCGGCTTTGCGGTCGATGGGCTTGCGTTTTTCATA AACTCTTATGTATGTAACAGAATGTCATAATGCTATAAACGCATCTTATA CAACTTTCAACAACGGATCTCTTGGCTCTCGCATCGATGAAGAACGCA GCGAAATGCGATAAGTAATGTGAATTGCAGAATTCAGTGAATCATCGAA TCTTTGAACGCACCTTGCGCCCCTTGGTATTCCGAGGGGCACGCCTGTT CGAGTGTCGTGAAATTCTCAACTCAATCCTCTTGTTATGAGAGGGCTGG GCTTGGACTTGGAGGTCTTGCCGGTGCTCCCTCGGGAAGTCGGCTCCT CTTGAATGCATGAGTGGATCCCTTTTGTAGGGTTTGCCCTTGGTGTGAT AATTATCTACGCCGCGGGTAGCCTTGCGTTGGTCTGCTTCTAACCGTCT TCGGACAACTTTCATCTCAACTTGACCTCGAATCAGGCGGGACTACCC GCTGAACTTAAGCATATCA).
4. The *Hericium erinaceus* fungus according to Item 1, which produces ergothioneine in a fermentation broth, wherein the amount of ergothioneine in the fermentation broth is more than 41 mg/L, wherein the fermentation broth is produced from a fermentation medium comprising carbon source, nitrogen source, inorganic salts and vitamins.
5. A composition comprising a *Hericium erinaceus* fungus and a fermentation broth, the fermentation broth comprising ergothioneine, wherein the amount of ergothioneine in the fermentation broth is more than 41mg/L, and wherein the fermentation broth is produced from a fermentation medium comprising carbon source, nitrogen source, inorganic salts and vitamins.
6. The composition according to Item 5, wherein the *Hericium erinaceus* fungus is a *Hericium erinaceus* fugus according to any one of Items 1 to 4.
7. A screening method for a *Hericium erinaceus* fungus, comprising the steps:
   (1) placing a tissue block of a *Hericium erinaceus* fungus on a potato dextrose agar solid medium containing an antibiotic, culturing for 5-12 days at 23~28°C to obtain *Hericium erinaceus* mycelia; inoculating the *Hericium erinaceus* mycelia on a potato dextrose agar solid medium, culturing for 5-12 days at 23~28°C to obtain a *Hericium erinaceus* strain;
   (2) inoculating the *Hericium erinaceus* strain obtained in step (1) into a fermentation medium, culturing for 12-20 days at 23~26 °C, wherein a precursor substance is fed on day 7 to day 10 during the culture, to obtain a fermentation broth;
   (3) centrifuging the fermentation broth, removing the supernatant for filtration, detecting the amount of ergothioneine in the filtrate, and screening the strain for the production of ergothioneine;
      preferably, the screening method further comprises a step, after step (2), of soak extraction of ergothioneine from the mycelium cells to the outside of the cells.
8. The screening method according to Item 7, wherein the step of soak extraction of ergothioneine from the mycelium cells is performed by:
   homogenizing the fermentation broth of mycelium, and then heating, thereby extracting the ergothioneine from the mycelium cells to the outside of the cells; or
   collecting the mycelium by solid-liquid separation of mycelium fermentation broth, preparing a mycelium suspension by adding water, homogenizing and then heating, thereby extracting the ergothioneine from the mycelium cells to the outside of the cells.
9. The screening method for *Hericium erinaceus* fungus according to Item 7, characterized in that the antibiotic in step (1) comprises any one or a combination of two or more of penicillin, streptomycin and chloramphenicol.
10. The screening method for *Hericium erinaceus* fungus according to Item 7, characterized in that the precursor substance in step (2) comprises any one or a combination of two or more of cysteine, methionine and histidine.
11. The screening method for *Hericium erinaceus* fungus according to Item 7, characterized in that the fermentation medium comprises 20 to 60 g/L of carbon source, 10 to 30 g/L of nitrogen source, 2 to 5 g/L of inorganic salts, and 0.001~0.005 g/L vitamins.

The advantages of the present invention include: the *Hericium erinaceus* strain HT-3 CCTCC No: M 2018567 provided by the present invention is easy to be cultivated and has a high yield of ergothioneine;

The screening method for *Hericium erinaceus* fungus provided by the present invention is simple, easy to be operated, low-cost, and is appliable to a large-scale screen, and easy to screen out a high-yield fungus strain.

### DESCRIPTION OF THE DRAWINGS

Figure 1 is a photograph showing the morphological characteristics of the colony *of Hericium erinaceus* HT-3 CCTCC NO: M 2018567.
Figure 2 is a photomicrograph of mycelium of *Hericium erinaceus* HT-3 CCTCC NO: M 2018567.
Figure 3 shows the sequence of the 18s rRNA gene of *Hericium erinaceus* HT-3 CCTCC NO: M 2018567.
Figure 4 shows the sequence of ITS of *Hericium erinaceus* HT-3 CCTCC NO: M 2018567.

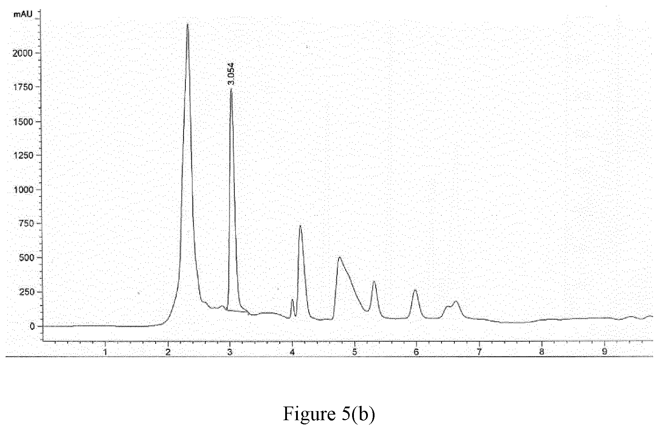

Figure 5 is HPLC chromatogram, showing the content of ergothioneine in fermentation broth of *Hericium erinaceus* HT-3 CCTCC NO: M 2018567 as determined by HPLC.

### DETAILED DESCRIPTION OF THE INVENTION

The following description of the present invention is only intended to illustrate specific embodiments for carrying out the present invention, and these embodiments should not be construed as limiting the present invention. Any other changes, modifications, substitutions, combinations and simplifications made without departing from the spirit and principle of the present invention are regarded as equivalents and fall within the protection scope of the present invention.

The experimental methods used below are conventional methods if no special requirements are indicated.

The materials and reagents used below are commercially available unless otherwise specified.

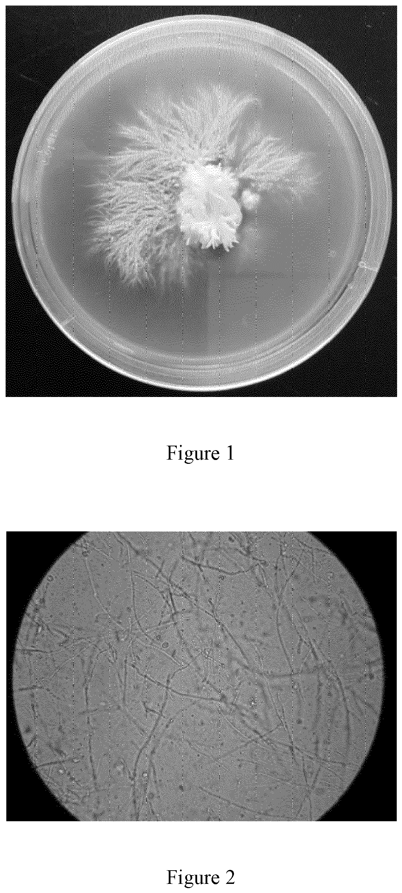

As a specific embodiment of the present invention, *Hericium erinaceus* HT-3 CCTCC No: M 2018567 grows quickly on potato dextrose agar medium (i.e., PDA solid medium). The colony after growing at 25°C for 8 days reaches 50-55 mm in diameter, and is white and velutinous, and extends radially; the back of the colony is light brown; see Figure 1 for the morphological characteristics of the colony. The hyphae of the strain are strong and thick, and composed of tubular cells with thin cell walls, and have septa and branches, and clamp connections, see Figure 2.

Lion's Mane Mushroom is *Hericium erinaceus* (Rull ex F.) Pers., Hydnaceae, Polyporaceae, Basidiomycetes, Fungi, and is a saprophytic and famous edible fungus. The whole shape resembles a hedgehog or hericium, so it is commonly named as hericium or Lion's Mane Mushroom. In the present invention, *Hericium erinaceus* is preferably *Hericium erinaceus* CCTCC No: M 2018567, which has been deposited at the China Center for Type Culture Collection (CCTCC) on August 23, 2018.

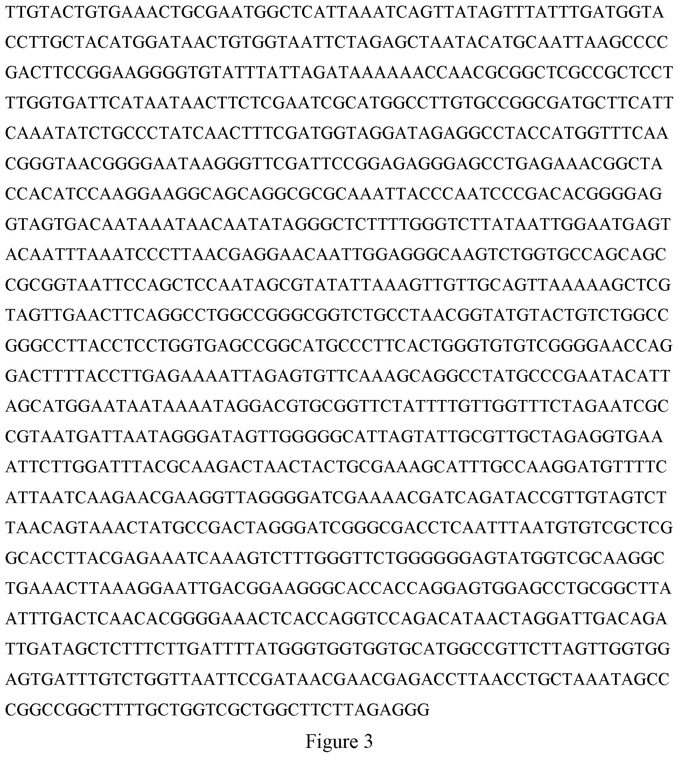

The 18s rRNA gene sequence of the *Hericium erinaceus* HT-3 CCTCC No: M2018567 is as shown in SEQ ID NO:1, see figure 3.

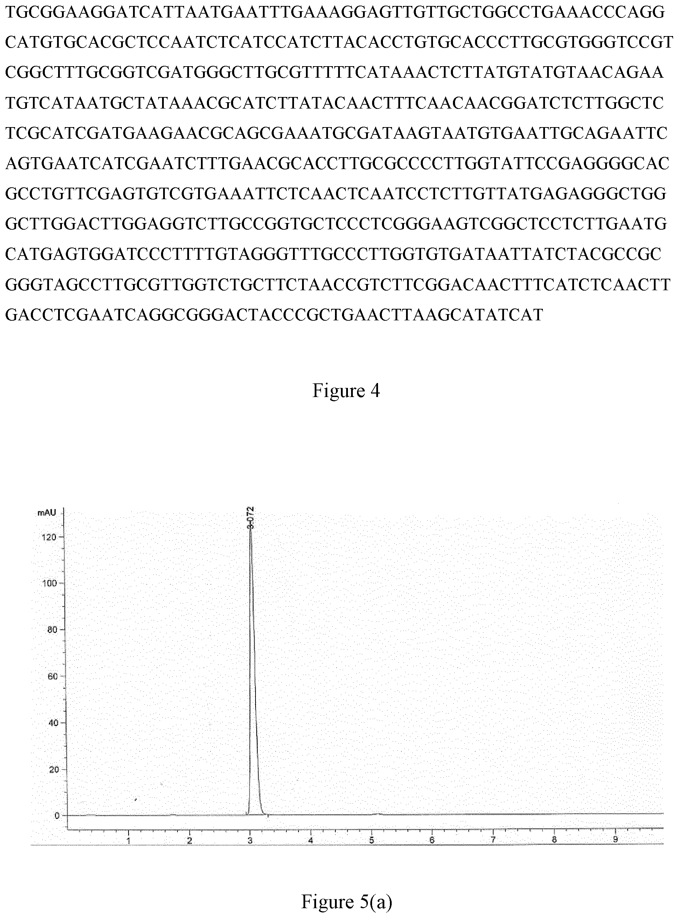

The ITS sequence of the *Hericium erinaceus* HT-3 CCTCC No: M 2018567 is as shown in SEQ ID NO:2, see figure 4.

As a specific embodiment of the present invention, the method for screening an ergothione-producing *Hericium* strain (the *Hericium erinaceus* HT-3) includes:
(1) cutting a tissue block of about 0.5 × 0.5 cm from *Hericium erinaceus* fungus and placing on a PDA solid medium containing an antibiotic, culturing for 5-12 days, preferably for 7-10 days, at 23~28 °C, preferably at 24°C, to obtain *Hericium erinaceus* mycelia; inoculating the *Hericium erinaceus* mycelia on a PDA solid medium, culturing for 5-12 days, preferably for 8 day, at 23~28°C, preferably at 24°C, to obtain a *Hericium erinaceus* strain;
   wherein the PDA solid medium, as a conventional solid medium, is a semi-synthetic medium and formulated as follows: a leach solution prepared from 200 g potato, 20 g glucose, 15-20g agar and 1000 mL water, without adjusting pH.
(2) inoculating the *Hericium erinaceus* strain obtained in step (1) into a fermentation medium, culturing for 12-20 days, preferably for 15 day, at 23~26 °C, preferably at 24°C, wherein a precursor substance is fed on day 7 to day 10 during the culture, to obtain a fermentation broth;
(3) centrifuging the fermentation broth at a speed of 8000~10000 rpm, preferably 8000 rpm, for 10~20 min, preferably for 20 min, removing the supernatant for filtration via a 0.22 µm microporous filter, detecting the amount of ergothioneine in the filtrate, and screening the strain for the production of ergothioneine. The tissue block from *Hericium erinaceus* fungus in step (1) refers to pileus, the junction of pileus and stipe, the middle part and the base of stipe, preferably pileus.

The antibiotic in step (1) comprises any one or a combination of two or more of penicillin, streptomycin and chloramphenicol, preferably streptomycin.

The concentration of the antibiotic is 0.01~0.1 g/L.

The precursor substance in the step (2) comprises any one or a combination of two or more of cysteine, methionine and histidine.

The concentration of each of the precursor substances in step (2) is 1~3 mM respectively.

The detection of the amount of ergothioneine in the filtrate in step (3) is performed by high performance liquid chromatography. HPLC conditions may be: Chromatographic column: Hypersil ODS C18 column (250 mm×4.6 mm, particle size 5 µm); column temperature: 30 °C; mobile phase: acetonitrile-water (3 : 97); flow rate: 1.0 mL/min; detection wavelength: 254 nm; loading volume: 20 µL.

In a specific embodiment, the screening method further comprises a step, after step (2), of soak extraction of ergothioneine from the mycelium cells to the outside of the cells. In a specific embodiment, the step of soak extraction of ergothioneine from the mycelium cells to the outside of the cells is performed by: homogenizing the fermentation broth of mycelium at 1000 to 6000 rpm, preferably at 4000 rpm, for 20 to 100 min, preferably for 60 min, and then raising the temperature of the fermentation broth to 60~100 °C , preferably 80 °C , and heating for 20~100 min, preferably for 50 min, thereby extracting the ergothioneine from the mycelium cells to the outside of the cells.

In a specific embodiment, the step of soak extraction of ergothioneine from the mycelium cells is performed as follows: collecting the mycelia by solid-liquid separation of mycelium fermentation broth, preparing a mycelium suspension by adding water, followed by homogenizing at1000 to 6000 rpm, preferably at 4000 rpm, for 20 to 100 min, preferably for 60 min, then raising the temperature of the fermentation broth to 60~100 °C , preferably 80 °C , and heating for 20~100 min, preferably for 60 min, thereby extracting the ergothioneine from the mycelium cells to the outside of the cells.

The fermentation medium comprises 20 to 60 g/L of carbon source, 10 to 30 g/L of nitrogen source, 2 to 5 g/L of inorganic salts, and 0.001~0.005 g/L vitamins.

Further, the carbon source comprised in the fermentation medium comprises any one or a combination of at least two of soluble starch, glucose, sucrose, fructose, maltose, and corn flour;
preferably, the carbon source comprised in the fermentation medium comprises any one or a combination of two of glucose and maltose.

The nitrogen source comprised in the fermentation medium comprises any one or a combination of at least two of tryptone, yeast powder, soybean meal, bran, soybean peptide powder, wheat peptone, casein peptone, corn steep liquor, beef extract and ammonium sulfate.

Preferably, the nitrogen source comprised in the fermentation medium comprises any one or a combination of the two of beef extract and tryptone.

The inorganic salt comprised in the fermentation medium comprises any one or a combination of at least two of NaH₂PO₄, K₂HPO₄, KH₂PO₄ and MgSO₄.

Preferably, the inorganic salt comprised in the fermentation medium is NaH₂PO₄.

The vitamins comprised in the fermentation medium comprise any one or a combination of at least two of vitamin B₁, vitamin B2, niacin, pantothenic acid, vitamin B₆, vitamin H and vitamin B₁₂;
preferably, the vitamins comprised in the fermentation medium comprise any one or a combination of the two of vitamin B₁ and niacin.

The present invention also relates to a composition comprising *Hericium erinaceus* HT-3 fermentation broth, said composition containing ergothioneine, wherein the amount of the ergothioneine in the fermentation broth is at least 41 mg/L, preferably at least 60 mg/L, more preferably at least 70 mg/L, still more preferably at least 80 mg/L, most preferably 90 mg/L, 100 mg/L, 110 mg/L, 120 mg/L, 130 mg/L, 140 mg/L or 150 mg/L. The *Hericium erinaceus* HT-3 is the *Hericium erinaceus* HT-3 CCTCC No: M 2018567 of the present invention.

### EXAMPLES

### EXAMPLE 1:

Screening of a *Hericium erinaceus* strain for the production of ergothioneine
(1) The tissue blocks of about 0.5×0.5cm were excised from the pilei of *Hericium erinaceus* fruiting bodies produced in different areas, placed on a PDA solid medium containing antibiotics, cultured at 24°C, and the resulting mycelia were inoculated onto a PDA solid medium for purification culture. The purified strains were inoculated on a PDA slant, stored at 3~4°C for later use;
(2) The solid strains obtained from step (1) were inoculated into the fermentation medium, and cultivated at 24°C for 16 days to obtain the mycelium fermentation broth. The fermentation medium was composed of 35 g/L sucrose , 20 g/L corn steep liquor, 3 g/L KH₂PO₄, 3 mg/L vitamin B₁ and water. After 10 days of fermentation, the precursor amino acids, cysteine, methionine and histidine, were fed, each at a concentration of 2 mM respectively.
(3) At the end of the fermentation, the mycelium fermentation broth was homogenized at 4000 rpm for 60 min, then the homogenized fermentation broth was heated to 80°C for 50 min, the ergothioneine was sock extracted from the mycelium cells into the fermentation broth, centrifuged at 8000 rpm for 20 min, the supernatant was removed for filtration through a 0.22 µm microporous filter, and the strain with the highest ergothioneine content in the filtrate was selected and named as *Hericium erinaceus* HT-3.

### EXAMPLE 2:

### Morphological observation of Hericium erinaceus HT-3

The *Hericium erinaceus* HT-3 CCTCC NO: M 2018567 grew quickly on potato dextrose agar medium, being adherent to and spreading from the surface of the medium. After growing at 25°C for 8 days, the colony reached 50-55 mm in diameter, being white and velvety, and extending radially to the surrounding, with a light brown back. The morphological characteristics of the colony can be seen in Figure 1. The hyphae of the strain are strong and thick, and composed of tubular cells with thin cell walls, and have septa and branches, and clamp connections, see Figure 2.

### EXAMPLE 3:

### Identification and classification of Hericium erinaceus HT-3

The *Hericium erinaceus* HT-3 was identified by genome sequencing by Shanghai Shenggong Biological Engineering (Shanghai) Co., Ltd. The strain was identified as *Hericium erinaceus,* and its taxonomy was: Fungi, Dikarya, Basidiomycota, Agaricomycotina, Agaricomycetidae, Russulales, Hericiaceae, Hericium. The result of 18s rRNA gene sequencing is shown in Figure 3, and the result of ITS sequencing is shown in Figure 4.

### EXAMPLE 4:

### Detection of ergothioneine

The HT-3 fermentation broth in Example 1 was used as the test sample, and the content of ergothioneine was determined by high performance liquid chromatography. HPLC conditions: Chromatographic column: Hypersil ODS C18 column (250 mm×4.6 mm, particle size 5 µm); column temperature: 30 °C; mobile phase: acetonitrile-water (3:97); flow rate: 1.0 mL/min; detection wavelength: 254 nm; loading volume: 20 µL. The chromatogram is shown in Figure 4, where a) is the standard product (the concentration of ergothioneine is 9.4 µg/mL), and b) is the HT-3 fermentation broth.

### EXAMPLE 5:

### Optimization of the fermentation medium

In this example, five fermentation media with different components, contents and pH values were selected for the experiment. The composition and pH of each fermentation medium are shown in Table 1 below.
(1) A slant of *Hericium erinaceus* CCTCC NO: M 2018567 mycelia were inoculated into liquid seed medium, and cultivated for 5-10 days at 15~30°C, at 100~300 rpm.
   Liquid seed medium: 4% (w/v) sucrose, 1.5% (w/v) soybean meal powder, 0.2% (w/v) sodium dihydrogen phosphate, 0.1% (w/v) sodium sulfate and adding water to 100%.
(2) The seed broth obtained in step (1) was inoculated into the fermentation medium for fermentation with the precursor substances added, and cultivated for 7-15 days at 15~30°C, at 100~300 rpm. At the end of fermentation the fermentation broth was recovered. The precursor amino acids, cysteine, methionine and histidine, each at a concentration of 2 mM, was supplemented on day 10 of the fermentation.
(3) At the end of the fermentation, the mycelium fermentation broth was homogenized at 4000 rpm for 60 min, then the homogenized fermentation broth was heated to 80°C for 50 min, the ergothioneine was extracted from the mycelium cells into the extracellular fermentation broth, centrifuged at 8000 rpm for 20 min, the supernatant was removed for filtration through a 0.22 µm microporous filter, and the ergothioneine content in the filtrate was determined.

Five different fermentation media were selected, and the ergothioneine contents measured in the final filtrate are shown in Table 1.

**Table 1**

| Serial numb er | Carbon source | Nitrogen source | Inorganic salts | Vitamins | pH value | Ergothioneine content |
|---|---|---|---|---|---|---|
| 1 | Sucrose 35 g/L | Corn steep liquor 20 g/L | KH₂PO₄ 3 g/L | Vitamin B₁ 3 mg/L | 4.5 | 132.1 mg/L |
| 2 | Soluble starch 20 g/L | Tryptone 30 g/L | KH₂PO₄ 5 g/L | Vitamin B₆ 5 mg/L | 5.2 | 41.2 mg/L |
| 3 | Glucose 60 g/L | Beef extract 10 g/L | K₂HPO₄ 3 g/L | Vitamin H 1 mg/L | 5.0 | 150.3 mg/L |
| 4 | Maltose 40 g/L | Casein peptone 25 g/L | KH₂PO₄ 2 g/L | Niacin 2 mg/L | 5.5 | 115.4 mg/L |
| 5 | Fructose 40 g/L | Wheat peptone 25 g/L | NaH₂PO₄ 2 g/L | Vitamin B₁₂ 2 mg/L | 5.1 | 83.2 mg/L |

At present, there have been reported some methods for preparing ergothioneine by biological fermentation. For example, ergothione can be produced, for example, by cultivation of *Pleurotus eryngii* mycelium in deep-liquid culture, with the yield at the end of fermentation reaching 62.20 mg/L; by cultivating *Lentinus edodes* mycelium in deep-liquid culture, with the yield at the end of fermentation reaching 23.6 mg/L; by liquid fermentation of *Lepista sordida,* with the maximum yield of 51 mg/L; by liquid fermentation of *Pleurotus plumonarius,* with the maximum yield of 48 mg/L; by liquid fermentation of wild *Pleurotus sapidus,* with the maximum yield of 37 mg/L. It can be seen that the yields of ergothioneine prepared by those biological fermentation methods are not high. However, using the strain according to the present invention, a higher yield of ergothioneine can be obtained under the condition of slightly optimized medium.

## Claims

1. A *Hericium erinaceus* fungus, with deposit number CCTCC No: M 2018567.

2. The *Hericium erinaceus* fungus according to claim 1, which has an 18s rRNA gene sequence as shown in SEQ ID NO:1.

3. The *Hericium erinaceus* fungus according to claim 1, which has an ITS sequence as shown in SEQ ID NO: 2.

4. The *Hericium erinaceus* fungus according to claim 1, which produces more than 41 mg/L ergothioneine in a fermentation broth, wherein the fermentation broth is produced from a fermentation medium comprising carbon source, nitrogen source, inorganic salts and vitamins.

5. A composition comprising a *Hericium erinaceus* fungus and a fermentation broth, the fermentation broth comprising ergothioneine, wherein the amount of ergothioneine in the fermentation broth is more than 41mg/L, and wherein the fermentation broth is produced from a fermentation medium comprising carbon source, nitrogen source, inorganic salts and vitamins.

6. The composition according to claim 5, wherein the *Hericium erinaceus* fungus is a *Hericium erinaceus* fugus according to any one of claims 1 to 4.

7. A screening method for a *Hericium erinaceus* fungus, comprising the steps:
(1) placing a tissue block of a *Hericium erinaceus* fungus on a potato dextrose agar solid medium containing an antibiotic, culturing for 5-12 days at 23~28°C to obtain *Hericium erinaceus* mycelia; inoculating the *Hericium erinaceus* mycelia on a potato dextrose agar solid medium, culturing for 5-12 days at 23~28°C to obtain a *Hericium erinaceus* strain;
(2) inoculating the *Hericium erinaceus* strain obtained in step (1) into a fermentation medium, culturing for 12-20 days at 23~26 °C, wherein a precursor substance is fed on day 7 to day 10 during the culture, to obtain a fermentation broth;
(3) centrifuging the fermentation broth, removing the supernatant for filtration, detecting the amount of ergothioneine in the filtrate, and screening the strain for the production of ergothioneine;
preferably, the screening method further comprises a step, after step (2), of soak extraction of intracellular ergothioneine from the mycelium cells to the outside of the cells.

8. The screening method according to claim 7, wherein the step of soak extraction of ergothioneine from the mycelium cells is performed by:
homogenizing the fermentation broth of mycelium, and then heating, thereby extracting the ergothioneine from the mycelium cells to the outside of the cells; or
collecting the mycelium by solid-liquid separation of mycelium fermentation broth, preparing a mycelium suspension by adding water, homogenizing and then heating, thereby extracting the ergothioneine from the mycelium cells to the outside of the cells.

9. The screening method for *Hericium erinaceus* fungus according to claim 7, wherein the antibiotic in step (1) comprises any one or a combination of two or more of penicillin, streptomycin and chloramphenicol.

10. The screening method for *Hericium erinaceus* fungus according to claim 7, wherein the precursor substance in step (2) comprises any one or a combination of two or more of cysteine, methionine and histidine.

11. The screening method for *Hericium erinaceus* fungus according to claim 7, wherein the fermentation medium comprises 20 to 60 g/L of carbon source, 10 to 30 g/L of nitrogen source, 2 to 5 g/L of inorganic salts, and 0.001~0.005 g/L vitamins.
